# EUROPÄISCHE PATENTSCHRIFT

(11) **EP 2 486 000 B1**
(45) Veröffentlichungstag und Bekanntmachung des Hinweises auf die Patenterteilung: **11.06.2014**
(21) Anmeldenummer: 10757166.3
(22) Anmeldetag: 17.09.2010
(51) Int. Cl.: C07C 67/08, C07C 69/28

(54) **VERFAHREN ZUR HERSTELLUNG VON POLYOLESTERN**
PROCESS FOR PREPARING POLYOL ESTERS
PROCÉDÉ DE PRODUCTION DE POLYOLESTERS

(30) Priorität: 08.10.2009 DE 102009048775
(43) Veröffentlichungstag der Anmeldung: 15.08.2012
(73) Patentinhaber: OXEA GmbH, 46147 Oberhausen (DE)
(72) Erfinder: FREY, Guido D., 64560 Riedstadt (DE); STRUTZ, Heinz, 47445 Moers (DE)
(86) Internationale Anmeldenummer: PCT/EP2010/005728
(87) Internationale Veröffentlichungsnummer: WO 2011/042116

(56) Entgegenhaltungen:
- EP-A2- 0 356 255
- US-A- 4 526 725
- US-A- 5 324 853

## Beschreibung

Die Erfindung betrifft ein Verfahren zur Herstellung von Polyolestern aus linearen oder verzweigten aliphatischen Monocarbonsäuren mit 3 bis 20 Kohlenstoffatomen und Polyolen durch Umsetzung der Ausgangsverbindungen in Gegenwart einer Lewissäure enthaltend mindestens ein Element der Gruppen 4 bis 14 des Periodensystems der Elemente als Katalysator.

Ester mehrwertiger Alkohole, auch Polyolester genannt, finden in großem Umfang und in vielfältiger Weise Anwendung in der Technik, wie zum Beispiel als Weichmacher oder Schmiermittel. Durch die Auswahl geeigneter Ausgangsprodukte lassen sich die physikalischen Stoffeigenschaften, wie zum Beispiel Siedepunkt oder Viskosität gezielt einstellen, und den chemischen Eigenschaften, wie der Hydrolyseresistenz oder der Stabilität gegenüber einem oxidativen Abbau Rechnung tragen. Auch auf die Lösung konkreter anwendungstechnischer Probleme können Polyolester gezielt zugeschnitten werden. Ausführliche Übersichten über den Einsatz von Polyolestern finden sich zum Beispiel in Ullmann's Encyclopedia of Industrial Chemistry, 5. Auflage, 1985, VCH Verlagsgesellschaft, Vol. A1, Seiten 305-319; 1990, Vol. A15, Seiten 438-440 oder in Kirk Othmer, Encyclopedia of Chemical Technology, 3. Auflage, John Wiley & Sons, 1978, Vol. 1, Seiten 778-787; 1981, Vol. 14, Seiten 496-498.
Die Verwendung von Polyolestern als Schmierstoffe besitzt eine große technische Bedeutung und sie werden besonders für solche Anwendungsgebiete eingesetzt, in denen Schmierstoffe auf Mineralölbasis die gesetzten Anforderungen nur unvollständig erfüllen. Polyolester werden insbesondere als Turbinenmotoren- und Instrumentenöle benutzt. Polyolester für Schmiermittelanwendungen basieren häufig auf 1,3-Propandiol, 1,3-Butandiol, 1,4-Butandiol, 1,2-Hexandiol, 1,6-Hexandiol, Neopentylglykol, Trimethylolpropan, Pentaerythrit, 2,2,4-Trimethylpentan-1,3-diol, Glycerin oder 3(4),8(9)-Dihydroxymethyltricyclo[5.2.1.0^{2.6}]decan, auch als TCD-Alkohol DM bezeichnet, als Alkoholkomponente.

Auch als Weichmacher werden Polyolester in erheblichem Umfang verwendet. Weichmacher finden in vielfältiger Weise Anwendung in Kunststoffen, Beschichtungsmitteln, Dichtungsmassen, Kautschuk- und Gummiartikeln. Sie treten mit hochpolymeren thermoplastischen Stoffen, ohne chemisch zu reagieren, vorzugsweise durch ihre Löse- und Quellvermögen in physikalische Wechselwirkung. Hierdurch bildet sich ein homogenes System aus, dessen thermoplastischer Bereich gegenüber den ursprünglichen Polymeren zu niederen Temperaturen verschoben ist, u. a. mit dem Ergebnis, dass seine mechanischen Eigenschaften optimiert, z. B. Formveränderungsvermögen, Elastizität, Festigkeit erhöht werden und die Härte verringert wird.

Um Weichmachern möglichst weite Anwendungsgebiete zu eröffnen, müssen sie eine Reihe von Kriterien erfüllen. Im Idealfall sollten sie geruchlos, farblos, licht-, kälte- und wärmebeständig sein. Überdies erwartet man, dass sie unempfindlich gegenüber Wasser, schwer brennbar und wenig flüchtig sind und die Gesundheit nicht schädigen. Weiterhin soll die Herstellung der Weichmacher einfach sein und, um ökologischen Anforderungen zu genügen, unter Vermeidung von Abfallstoffen, wie nicht weiterverwertbaren Nebenprodukten und Schadstoffe enthaltenden Abwässern, erfolgen.

Eine spezielle Klasse von Polyolestern (sie werden kurz als G-Ester bezeichnet) enthält als Alkoholkomponente Diole bzw. Etherdiole, beispielsweise Ethylenglykol, Diethylenglykol, Triethylenglykol, Tetraethylenglykol, 1,2-Propylenglykol oder höhere Propylenglykole. Ihre Herstellung kann auf unterschiedlichen Wegen erfolgen. Neben der Umsetzung von Alkohol und Säure gegebenenfalls in Gegenwart saurer Katalysatoren werden in der Praxis weitere Prozesse zur Gewinnung von G-Estern angewandt, u. a. die Reaktion von Diol mit Säurehalogenid, die Umesterung eines Carbonsäureesters mit einem Diol und die Addition von Ethylenoxid an Carbonsäuren (Ethoxylierung). In der industriellen Fertigung haben sich lediglich die unmittelbare Umsetzung von Diol und Carbonsäure und die Ethoxylierung von Carbonsäuren als Produktionsverfahren durchgesetzt, wobei der Veresterung von Diol und Säure zumeist der Vorzug gegeben wird. Denn dieses Verfahren kann ohne besonderen Aufwand in konventionellen chemischen Apparaten durchgeführt werden und es liefert chemisch einheitliche Produkte. Demgegenüber erfordert die Ethoxylierung umfangreiche und kostenintensive technische Mittel. Ethylenoxid ist eine sehr reaktive chemische Substanz. Es kann explosionsartig polymerisieren und bildet mit Luft in sehr weiten Mischungsbereichen explosive Gemische. Ethylenoxid reizt die Augen und Atemwege, führt zu Verätzungen, zu Leber- und Nierenschäden und ist karzinogen. Seine Handhabung erfordert daher umfangreiche Sicherheitsmaßnahmen. Überdies ist auf peinliche Sauberkeit von Lagervorrichtungen und Reaktionsapparaten zu achten, um die Bildung unerwünschter Verunreinigungen durch Nebenreaktionen des Ethylenoxids mit Fremdstoffen auszuschließen. Schließlich ist die Reaktion mit Ethylenoxid nicht sehr selektiv, denn sie führt zu Gemischen von Verbindungen unterschiedlicher Kettenlänge.

Die unmittelbare Veresterung von Alkoholen mit Carbonsäuren gehört zu den Grundoperationen der organischen Chemie. Um die Reaktionsgeschwindigkeit zu erhöhen, führt man die Umsetzung üblicherweise in Gegenwart von Katalysatoren durch. Der Einsatz eines der Reaktanten im Überschuss und/oder die Abtrennung des im Verlauf der Reaktion gebildeten Wassers stellt sicher, dass das Gleichgewicht entsprechend dem Massenwirkungsgesetz zur Seite des Reaktionsproduktes, also des Esters, verschoben wird, d. h. hohe Ausbeuten erzielt werden.

Umfassende Angaben zur Herstellung von Estern mehrwertiger Alkohole, darunter auch Estern aus Ethylenglykolen und Fettsäuren und zu den Eigenschaften ausgewählter Vertreter dieser Verbindungsklassen finden sich in Goldsmith, Polyhydric Alcohol Esters of Fatty Acids, Chem. Rev. 33, 257 ff. (1943). Beispielsweise erfolgt die Herstellung von Estern des Diethylenglykols, des Triethylenglykols und von Polyethylenglykolen über Reaktionszeiten von 2,5 bis 8 Stunden bei Temperaturen von 130 bis 230°C. Zur Entfernung des Reaktionswassers verwendet man Kohlendioxid. Als geeignete Katalysatoren für die Veresterung mehrwertiger Alkohole werden anorganische Säuren, saure Salze, organische Sulfonsäuren, Acetylchlorid, Metalle oder amphotere Metalloxide genannt. Die Entfernung des Reaktionswassers erfolgt mit Hilfe eines Schleppmittels, beispielsweise Toluol oder Xylol oder durch Einleiten inerter Gase wie Kohlendioxid oder Stickstoff.

Auf die Gewinnung und die Eigenschaften von Fettsäureestern der Polyethylenglykole geht Johnson (Edit.), Fatty Acids in Industry (1989) Kap. 9, Polyoxyethylene Esters of Fatty Acid ein und gibt eine Reihe präparativer Hinweise. Höhere Diesterkonzentrationen erzielt man durch die Erhöhung des molaren Verhältnisses von Carbonsäure zu Glykol. Geeignete Maßnahmen zur Entfernung des Reaktionswassers sind die Azeotropdestillation in Gegenwart eines mit Wasser nicht mischbaren Lösungsmittels, das Erhitzen unter Durchleiten eines Inertgases oder die Durchführung der Reaktion unter Vakuum in Gegenwart eines Trockenmittels. Verzichtet man auf den Zusatz von Katalysatoren, so sind längere Reaktionszeiten und höhere Reaktionstemperaturen erforderlich. Beide Reaktionsbedingungen können durch den Einsatz von Katalysatoren gemildert werden. Neben Schwefelsäure sind organische Säuren wie p-Toluolsulfonsäure sowie Kationenaustauscher vom Polystyroltyp die bevorzugten Katalysatoren. Auch die Verwendung von Metallpulvern, wie Zinn oder Eisen wird beschrieben. Nach der Lehre von US 2,628,249 lassen sich Farbprobleme bei der Katalyse mit Schwefelsäure oder Sulfonsäure mildern, wenn in Gegenwart von Aktivkohle gearbeitet wird.

Als weitere metallhaltige Katalysatoren werden auch Titan-, Zirconium- oder Zinnalkoholate, -carboxylate oder -chelate zur Herstellung von Polyolestern, beispielsweise gemäß US 5,324,853 A1, eingesetzt. Solche Metallkatalysatoren kann man als Hochtemperaturkatalysatoren ansehen, denn sie erreichen ihre volle Aktivität erst bei hohen Veresterungstemperaturen, im Allgemeinen oberhalb von 180°C. Sie werden häufig nicht zu Beginn der Veresterungsreaktion zugesetzt sondern nachdem das Reaktionsgemisch bereits aufgeheizt wurde und zum Teil unter Wasserabspaltung reagiert hat. Trotz der im Vergleich zur klassischen Schwefelsäurekatalyse erforderlichen höheren Reaktionstemperaturen und längeren Reaktionszeiten werden bei der Katalyse mit solchen metallhaltigen Verbindungen Rohester mit einer vergleichsweise geringen Farbzahl erhalten. Gängige Veresterungskatalysatoren sind beispielsweise Tetra(isopropyl)orthotitanat, Tetra(butyl)orthotitanat, Tetra(butyl)zirconat oder Zinn(II)-2-ethylhexanoat.

Bei der katalytischen Veresterungsreaktion von Polyolen mit Carbonsäuren wird, bezogen auf die im Unterschuss vorliegende Komponente, innerhalb einer vergleichsweise kurzen Zeit ein hoher Umsatz erzielt, doch für den Restumsatz zu den gewünschten Polyolestern muss noch eine verhältnismäßig lange Reaktionsdauer in Kauf genommen werden. Zwar erhält man dann einen Polyolester mit einem akzeptablen Restgehalt an teilveresterten Produkten, ausgedrückt durch die Hydroxylzahl in mg KOH/g (gemäß DIN 53240) oder durch den gaschromatographisch ermittelten Gehalt an teilveresterten Produkten, doch lange Reaktionszeiten sind wirtschaftlich nachteilig, da sie die Leistung der technischen Produktionsanlage begrenzen. Um auch den Restumsatz zu beschleunigen, schlägt US 5,324,853 A1 eine intensive Durchmischung des Reaktionsansatzes vor.
Nach beendeter Veresterungsreaktion ist eine ausreichende Abtrennung des Metallkatalysators sicherzustellen, da Metallspuren in den aufgereinigten Polyolestern ihre Anwendung als Weichmacher oder Schmiermittel beeinträchtigen können, indem beispielsweise die elektrische Leitfähigkeit oder die Stabilität gegenüber Luftsauerstoff beeinflusst wird. Gemäß der Arbeitsweise aus US 5,324,853 A1 versetzt man das rohe Veresterungsgemisch mit einer wässrigen Sodalösung und gegebenenfalls mit Aktivkohle. Durch diese Arbeitsweise werden die Metallverbindungen zu unlöslichen Feststoffen hydrolysiert und können vor der weiteren Aufarbeitung der rohen Esterverbindung abfiltriert werden. Nach US 4,304,925 A1 wird das rohe Veresterungsprodukt vor Alkalizugabe zunächst mit Wasser versetzt und in der Wärme behandelt. Dadurch werden die hydrolysierten Metallverbindungen in gut filtrierbare Niederschläge überführt.

EP 0 356 255 A2 behandelt die Veresterung von propoxyliertem Glycerin und gesättigten oder ungesättigten C₁₀-C₂₄-Fettsäuren in Gegenwart von Zinn- und Titanatkatalysatoren. Der Rohester wird mit Natronlauge behandelt und anschließend in Gegenwart eines Filtrierhilfsmittels filtriert.

US 4,526,725 A1 offenbart chelatisierte Alkyltitanatkatalysatoren und ihre Verwendung als Veresterungskatalysator. Der Rohester wird zur Entfernung von Alkoholresten einer Wasserdampfdestillation unterzogen.

Der Stand der Technik zur Herstellung von Polyolestern unter Metallkatalyse erfordert entweder eine besondere Reaktorauslegung, um die Veresterungsreaktion in einer wirtschaftlich vertretbaren Zeit zu vervollständigen, oder eine zusätzliche Behandlung mit Wasser in der Wärme, um den metallhaltigen Katalysator nach beendeter Veresterungsreaktion unter Bildung gut abfiltrierbarer Hydrolyseprodukte möglichst vollständig zu entfernen.

Es bestand daher die Aufgabe, die bekannten Verfahren zu verbessern und durch Abstimmung und Vereinfachung der aufeinanderfolgenden Teilschritte des gesamten Prozesses das Verfahren zu optimieren und die Gewinnung von Polyolestern in hoher Qualität zu vereinfachen, so dass Polyolester möglichst vielseitig angewendet werden können.

Die Erfindung besteht daher in einem Verfahren zur Herstellung von Polyolestern durch Umsetzung von Polyolen mit linearen oder verzweigten aliphatischen Monocarbonsäuren mit 3 bis 20 Kohlenstoffatomen, dadurch gekennzeichnet, dass man eine Mischung der Ausgangsverbindungen in Gegenwart einer Lewissäure enthaltend mindestens ein Element der Gruppen 4 bis 14 des Periodensystems der Elemente als Katalysator unter Entfernung des gebildeten Wassers reagieren lässt, wobei die Umsetzung der Ausgangsverbindungen in Gegenwart eines Adsorbens erfolgt, und anschließend eine Wasserdampfbehandlung zur Restsäure- und Wasserentfernung durchführt.

Die Reaktion zwischen den Ausgangsverbindungen Polyol und der aliphatischen Monocarbonsäure setzt in Abhängigkeit von den Einsatzmaterialien im Bereich von etwa 120 bis 180°C ein und kann anschließend auf unterschiedlich ausgestaltete Weise zu Ende geführt werden.

Nach einer Ausgestaltung des erfindungsgemäßen Verfahrens wird zunächst ausgehend von Raumtemperatur auf eine Temperatur bis auf maximal 280°C, vorzugsweise bis auf 250°C erhitzt und bei konstant gehaltener Temperatur der Druck ausgehend von Normaldruck stufenweise erniedrigt, um die Entfernung des Reaktionswassers zu erleichtern. Die Wahl der Druckstufen, ob ein-, zwei- oder mehrstufig, sowie der auf der jeweiligen Stufe einzustellende Druck kann über einen weiten Bereich variiert und den jeweiligen Bedingungen angepasst werden. Beispielsweise kann in einer ersten Stufe der Druck ausgehend von Normaldruck zunächst bis auf 600 hPa erniedrigt werden und anschließend die Reaktion bei einem Druck von 300 hPa zu Ende geführt werden. Bei diesen Druckangaben handelt es sich um Richtwerte, die zweckmäßig eingehalten werden.

Neben der Variation des Drucks kann ebenfalls auch die Temperatur ausgehend von Raumtemperatur während der Veresterungsreaktion ein-, zweioder mehrstufig verändert werden, so dass bei konstant eingestelltem Druck die Temperatur von Stufe zu Stufe erhöht wird, üblicherweise bis auf eine maximale Temperatur von 280°C. Es hat sich aber als zweckmäßig erwiesen, bei von Stufe zu Stufe ansteigender Temperatur auf maximal 280°C zu erhitzen und auch den Druck von Stufe zu Stufe zu erniedrigen. Beispielsweise kann die Veresterungsreaktion ausgehend von Raumtemperatur in einer ersten Stufe bei einer Temperatur bis auf 190°C geführt werden. Ebenfalls wird ein verminderter Druck bis auf 600 hPa angelegt, um das Austreiben des Reaktionswassers zu beschleunigen. Nach Erreichen der Temperaturstufe von 190°C wird der Druck nochmals bis auf 300 hPa erniedrigt und die Veresterungsreaktion bei einer Temperatur bis auf 250°C zu Ende geführt. Bei diesen Temperatur- und Druckangaben handelt es sich um Richtwerte, die zweckmäßiger Weise eingehalten werden. Die einzustellenden Temperatur- und Druckbedingungen auf den jeweiligen Stufen, die Zahl der Stufen sowie die jeweilige Temperaturerhöhungs- oder Druckminderungsrate pro Zeiteinheit können über einen weiten Bereich variiert werden und entsprechend den physikalischen Eigenschaften der Ausgangsverbindungen und der Reaktionsprodukte angepasst werden, wobei die Temperatur- und Druckbedingungen der ersten Stufe ausgehend von Normaldruck und Raumtemperatur eingestellt werden. Besonders zweckmäßig hat es sich erwiesen, die Temperatur in zwei Stufen zu erhöhen und den Druck in zwei Stufen zu erniedrigen.

Die untere Grenze des einzustellenden Drucks hängt von den physikalischen Eigenschaften, wie Siedepunkte und Dampfdrücke, der Ausgangsverbindungen sowie der gebildeten Reaktionsprodukte ab und wird auch durch die Anlagenausstattung festgelegt. Ausgehend von Normaldruck kann innerhalb dieser Grenzwerte stufenweise mit von Stufe zu Stufe abnehmenden Drücken gearbeitet werden. Die obere Temperaturgrenze, üblicherweise 280°C, ist einzuhalten, um die Bildung von Zersetzungsprodukten, die u. a. farbschädigend wirken, zu vermeiden. Die untere Grenze der Temperaturstufen wird durch die Reaktionsgeschwindigkeit bestimmt, die noch ausreichend hoch sein muss, um die Veresterungsreaktion innerhalb einer vertretbaren Zeit abzuschließen. Innerhalb dieser Grenzwerte kann stufenweise mit von Stufe zu Stufe ansteigenden Temperaturen gearbeitet werden.

Die jeweiligen Reaktionsbedingungen, wie Temperatur, Reaktionszeit, anzulegender Druck oder einzusetzender Katalysator sind auf den jeweiligen Polyolester zuzuschneiden, um bei einer ausreichenden Reaktionsgeschwindigkeit die Bildung farbgebender Komponenten in den Hintergrund zu drängen und Abbaureaktionen des Polyolesters möglichst zu vermeiden. Insbesondere bei Polyolestern auf Basis von Etherdiolen, wie zum Beispiel Triethylenglykol oder Tetraethylenglykol, kann ein verstärkter Abbau des Ethergerüstes einsetzen, wenn die Reaktionsbedingungen, wie Temperatur, Reaktionszeit und Katalysatorart und -menge, nicht gezielt auf den jeweiligen Polyolester eingestellt werden.

Die Veresterung kann mit stöchiometrischen Mengen Polyol und aliphatischer Monocarbonsäure vorgenommen werden. Vorzugsweise lässt man das Polyol jedoch mit überschüssiger Monocarbonsäure reagieren, die im Allgemeinen die leichter siedende Komponente ist und die bei der nachfolgenden Aufarbeitung des Rohesters auf einfache Weise destillativ abgetrennt werden kann. Die aliphatische Monocarbonsäure wird in einem 10 bis 50%-igen molaren, vorzugsweise in einem 20 bis 40 %-igen molaren Überschuss je Mol zu veresternder Hydroxylgruppe des Polyols eingesetzt.

Das gebildete Reaktionswasser wird im Laufe der Veresterungsreaktion zusammen mit der überschüssigen Monocarbonsäure aus dem Reaktionsgefäß abdestilliert und in einen nachgeschalteten Phasentrenner geleitet, in dem sich Monocarbonsäure und Wasser je nach ihren Löslichkeitseigenschaften auftrennen. Gegebenenfalls bildet die eingesetzte Monocarbonsäure mit Wasser unter den Reaktionsbedingungen auch ein Azeotrop und vermag als Schleppmittel das Reaktionswasser zu entfernen. Aus dem Wasseranfall kann der Reaktionsverlauf verfolgt werden. Das abgeschiedene Wasser wird aus dem Prozess entfernt während die Monocarbonsäure aus dem Phasentrenner wieder in das Reaktionsgefäß zurückfließt. Die Zugabe eines weiteren organischen Lösungsmittels, wie Hexan, 1-Hexen, Cyclohexan, Toluol, Xylol oder Xylolisomerengemische, das die Aufgabe des Azeotropbildners übernimmt, ist nicht ausgeschlossen, jedoch auf wenige Ausnahmefälle beschränkt. Der Azeotropbildner kann bereits zu Beginn der Veresterungsreaktion oder nach dem Erreichen höherer Temperaturen zugesetzt werden. Wenn die theoretisch zu erwartende Wassermenge angefallen ist oder die Hydroxylzahl, beispielsweise bestimmt nach DIN 53240, unter einen festgelegten Wert gefallen ist, beendet man die Reaktion indem man den Reaktionsansatz abkühlen lässt.

Als Katalysatoren für die Veresterung des Polyols mit der Monocarbonsäure verwendet man Lewissäuren enthaltend mindestens ein Elemente der Gruppen 4 bis 14 des Periodensystems der Elemente und die in fester oder flüssiger Form eingesetzt werden können. Unter dem Begriff Lewissäure im Sinne der Erfindung wird die allgemein übliche Definition für solche Elemente oder Verbindungen verstanden, die eine Elektronenlücke aufweisen, wie beispielsweise in Römpp's Chemie-Lexikon, 8. Auflage, Franck'sche Verlagshandlung 1983, Band 3, H-L ausgeführt. Zu den besonders geeigneten Lewissäuren, die als Katalysatoren in der Veresterungsreaktion eingesetzt werden können, zählen Titan, Zirkonium, Eisen, Zink, Bor, Aluminium oder Zinn, die als Element in fein verteilter Form oder bevorzugt in Form von Verbindungen verwendet werden. Geeignete Verbindungen sind beispielsweise Zinn(II)-oxid, Zinn(IV)oxid, Zinn-Carboxylate, wie Zinn(II)-2-ethylhexanoat, Zinn(II)-oxalat, Zinn(II)-acetat oder Zinn(IV)-acetat, Zinn(IV)-alkoholate, wie Tetra(methyl)stannat, Tetra(ethyl)stannat, Tetra(propyl)stannat, Tetra(isopropyl)stannat oder Tetra(isobutyl)stannat, oder Organozinnverbindungen, wie Butylzinnmaleat oder Dibutylzinndilaurat. Zu den geeigneten Titanverbindungen zählen Alkoholate, wie Tetra(methyl)orthotitanat, Tetra(ethyl)orthotitanat, Tetra(propyl)orthotitanat, Tetra(iso-propyl)orthotitanat, Tetra(butyl)orthotitanat, Tetra(iso-butyl)orthotitanat, Tetra(pentyl)orthotitanat oder Tetra(2-ethylhexyl)orthotitanat; Acylate, wie Hydroxytitanacetat, Hydroxytitanbutyrat oder Hydroxytitanpentanoat oder Chelate, wie Tetraethylenglykoltitanat oder Tetrapropylenglykoltitanat. Auch die entsprechenden Zirkoniumverbindungen können mit Erfolg eingesetzt werden, wie Tetra(methyl)orthozirkonat, Tetra(ethyl)orthozirkonat, Tetra(propyl)orthozirkonat, Tetra(iso-propyl)orthozirkonat, Tetra(butyl)orthozirkonat, Tetra(iso-butyl)orthozirkonat, Tetra(pentyl)orthozirkonat oder Tetra(2-ethylhexyl)orthozirkonat. Geeignet sind ebenfalls Borsäure sowie Borsäureester, wie Borsäuretrimethylester, Borsäuretriethylester, Borsäuretripropylester, Borsäuretriisopropylester, Borsäuretributylester oder Borsäuretriisobutylester.

Ebenfalls geeignet sind Aluminiumoxid, Aluminiumhydroxid, Aluminiumcarboxylate, wie Aluminiumacetat oder Aluminiumstearat, oder Aluminiumalkoholate wie Aluminiumtributylat, Aluminium-tri-sec.-butylat, Aluminium-tri-tert.-butylat oder Aluminium-tri-isopropylat.

Auch Zinkoxid, Zinksulfat und Zinkcarboxylate wie Zinkacetat Dihydrat oder Zinkstearat, und Eisen(II)acetat oder Eisen(III)hydroxid-oxid können als Katalysatoren eingesetzt werden.

Der Katalysator kann dem Reaktionsgemisch bereits zu Beginn zugesetzt werden oder erst nachträglich unter Beachtung von Sicherheitsmaßnahmen bei erhöhter Temperatur, wenn beispielsweise die Abtrennung des Reaktionswassers eingesetzt hat.

Die Menge des zugesetzten Veresterungskatalysators beträgt 1X10⁻⁵ bis 20 mol%, vorzugsweise 0,01 bis 5 mol-%, insbesondere 0,01 bis 2 mol%, bezogen auf die im Unterschuss zugesetzte Ausgangsverbindung, zweckmäßigerweise bezogen auf Polyol. Bei höheren Katalysatormengen ist mit Spaltungsreaktionen der Polyolester zu rechnen.

Besonders bei der Herstellung von Polyolestern auf Basis von Etherdiolen, wie zum Beispiel Triethylenglykol oder Tetraethylenglykol, ist bei dem Einsatz hoher Katalysatorkonzentrationen gegen Reaktionsende und in der Phase des Umsatzes letzter Reste an freien Hydroxylgruppen eine verstärkte Spaltung der Etherkette zu befürchten, so dass in diesem Falle die Reaktionstemperatur oder der anzulegende Druck anzupassen sind. Je höher die gewählte Katalysatorkonzentration ist, desto niedriger ist im Allgemeinen die Reaktionstemperatur oder der anzulegende Druck zu wählen und es ist nach einem optimierten Temperatur- und Druckprofil zu arbeiten. Bei zu niedrigen Katalysatorkonzentrationen wird die Veresterungsgeschwindigkeit so gering, dass kein akzeptabler Umsatz in einer vertretbaren Reaktionszeit beobachtet wird.

Die Zugabe des Veresterungskatalysators kann in flüssiger oder fester Form erfolgen. Feste Katalysatoren, beispielsweise Zinn(II)oxid, Zinkoxid oder Eisen(III)hydroxid-oxid werden nach beendeter Veresterungsreaktion abfiltriert, bevor der rohe Polyolester der weiteren Aufarbeitung unterzogen wird. Werden die Veresterungskatalysatoren als flüssige Verbindungen zugesetzt, beispielsweise Tetra(iso-propyl)orthotitanat oder Tetra(butyl)orthotitanat, die nach beendeter Veresterungsreaktion im Reaktionsgemisch noch gelöst vorliegen, so werden diese Verbindungen im Laufe des Aufarbeitungsverfahrens bei der Wasserdampfbehandlung in unlösliche, gut abfiltrierbare Niederschläge überführt.

Bei dem erfindungsgemäßen Verfahren wird die Veresterung in Gegenwart eines Adsorbens durchgeführt. Man verwendet dabei poröse, großflächige feste Materialien, die üblicherweise in der chemischen Praxis sowohl im Labor als auch in technischen Anlagen eingesetzt werden. Beispiele für solche Materialien sind oberflächenreiche Polykieselsäuren wie Silicagele (Kiesel-Xerogele), Kieselgel, Kieselguhr, oberflächenreiche Aluminiumoxide und Aluminiumoxidhydrate, mineralische Materialien, wie Tone oder Carbonate, oder Aktivkohle. Besondert bewährt hat sich Aktivkohle. Im Allgemeinen ist das Adsorbens feinteilig in der Reaktionslösung suspendiert, die durch intensives Rühren oder durch Einleiten eines Inertgases bewegt wird. Dadurch wird ein inniger Kontakt zwischen der flüssigen Phase und dem Adsorbens erreicht. Die Menge des Adsorbens kann weitgehend frei und somit den individuellen Erfordernissen entsprechend eingestellt werden. Bezogen auf 100 Gewichtsteile des flüssigen Reaktionsansatzes bewährt es sich 0,1 bis 5, vorzugsweise 0,1 bis 1,5 Gewichtsteile des Adsorbens einzusetzen.
Wegen der eingangs beschriebenen Qualitätskriterien für Polyolester sind die Verfahrensschritte bei der Veresterungsstufe unter Entfernung des Reaktionswassers und bei der Aufarbeitung des Rohesters sehr wesentliche Prozessmerkmale, denn die Abstimmung dieser Verfahrensschritte beeinflusst in wesentlichem Maße die sensorischen und optischen Eigenschaften der Endprodukte. Insbesondere werden durch eine optimierte Prozessführung Polyolester auf Basis von Etherdiolen, beispielsweise Triethylenglykol oder Tetraethylenglykol, mit hoher Reinheit sowie geringer Farbzahl und hoher Farbstabilität gewonnen. Die Struktur der Ausgangsstoffe, der mehrwertigen Alkohole und der aliphatischen Monocarbonsäuren, ist dagegen für die mechanischen und thermischen Eigenschaften der mit den Polyolestern weichgestellten Kunststoffmassen maßgebend und beeinflusst die Hydrolyse- und Oxidationsstabilität von Schmiermitteln.

Das nach Beendigung der Umsetzung anfallende Reaktionsgemisch enthält neben dem Polyolester als erwünschtem Reaktionsprodukt gegebenenfalls nicht umgesetzte Ausgangsstoffe, insbesondere noch überschüssige aliphatische Monocarbonsäure, sofern nach der bevorzugten Ausgestaltung des erfindungsgemäßen Verfahrens mit einem Monocarbonsäureüberschuss gearbeitet wird. Üblicherweise destilliert man zunächst unumgesetzte und im Überschuss vorliegende Ausgangsverbindungen ab, zweckmäßigerweise unter Anlegen eines verminderten Drucks.

Anschließend wird der Rohester einer Behandlung mit Wasserdampf unterzogen, die beispielsweise in einfacher Form durch Einleiten von Wasserdampf in das Rohprodukt erfolgen kann. Ein Vorteil der Wasserdampfbehandlung ist, dass in ihrem Verlauf noch vorhandener Katalysator zerstört und in gut abfiltrierbare Hydrolyseproduke überführt wird. Da die Veresterungsreaktion in Gegenwart eines Adsorbens durchgeführt wird, erleichtert das bereits anwesende Adsorbens die Abscheidung der Katalysatorfolgeprodukte. Die Anwesenheit eines Adsorbens während der Wasserdampfbehandlung wirkt sich ebenfalls vorteilhaft auf die Farbe und auf die Farbstabilität des Polyolesters aus. Es ist aber auch möglich, nach beendeter Veresterungsreaktion und Abtrennung überschüssiger Ausgangsverbindungen, also vor Durchführung der Wasserdampfdestillation, das Adsorbens abzufiltrieren.

Die Wasserdampfbehandlung wird im Allgemeinen bei Normaldruck durchgeführt, obwohl die Anwendung eines leichten Unterdrucks zweckmäßigerweise bis 400 hPa nicht ausgeschlossen ist. Die Wasserdampfbehandlung erfolgt im Allgemeinen bei Temperaturen von 100 bis 250°C, vorzugsweise von 150 bis 220°C und insbesondere von 170 bis 200°C und richtet sich auch nach den physikalischen Eigenschaften der jeweils herzustellenden Polyolester.

Bei dem Prozessschritt der Wasserdampfbehandlung erweist es sich als zweckmäßig, während der Aufheizperiode bis zum Erreichen der Arbeitstemperatur möglichst schonend vorzugehen, um den Rohester auf die erforderliche Temperatur für die Wasserdampfbehandlung zu erhitzen.

Die Dauer der Wasserdampfbehandlung lässt sich durch Routineversuche ermitteln und sie wird im Allgemeinen über einen Zeitraum von 0,5 bis 5 Stunden durchgeführt. Eine zu lange Wasserdampfbehandlung führt zu einer ungewünschten Erhöhung der Farbzahl des Polyolesters und ist daher zu vermeiden. Auch beobachtet man eine verstärkte Abbaureaktion des Polyolesters zu sauer reagierenden Verbindungen, deren Gehalt sich in einem Anstieg der Neutralisationszahl oder Säurezahl beispielsweise bestimmt nach DIN EN ISO 3682/ASTM D 1613 zeigt. Bei einer zu kurzen Behandlungsdauer ist die Restsäure- und Wasserentfernung nicht ausreichend wirksam und der gewünschte Polyolester weist noch eine zu hohe unerwünschte Säurezahl und einen zu hohen Wassergehalt auf. Auch wird bei zu kurzer Behandlungsdauer nur ein geringer vorteilhafter Effekt auf die Farbzahl des Polyolesters beobachtet.

Auch die Bedingungen der Wasserdampfbehandlung wie, Temperatur, Dauer und anzulegender Druck sind gezielt auf den jeweiligen Polyolester einzustellen, um ein optimales Ergebnis in Bezug auf die Farbzahl der Polyolester zu erzielen und um Restgehalte an Ausgangsverbindungen, Wasser und Katalysatorspuren möglichst zu minimieren und gleichzeitig Abbaureaktionen zu unterdrücken. Insbesondere bei der Anwendung höherer Katalysatormengen und bei der Herstellung von Polyolestern auf Basis von Etherdiolen, wie zum Beispiel Triethylenglykol oder Tetraethylenglykol, sind die Bedingungen bei der Wasserdampfbehandlung auf den jeweiligen Polyolestern genau zuzuschneiden, um den unerwünschten Abbau der Etherkette zu unterbinden.

Gegebenenfalls erfolgt nach der Wasserdampfbehandlung die Zugabe eines festen, alkalisch reagierenden Stoffes, beispielsweise basisches Siliziumdioxid, basisches Aluminiumoxid oder Natriumcarbonat, Natriumhydrogencarbonat, Calciumcarbonat, oder Natriumhydroxid in fester Form sowie basisch reagierende Mineralien, um die Neutralisationszahl des Polyolesters weiter zu verringern.

An die Wasserdampfbehandlung schließt sich, gegebenenfalls nach Filtration des Adsorbens, der gegebenenfalls zugesetzten festen, alkalisch reagierenden Stoffe und weiterer angefallener Feststoffe, die Trocknung des Polyolesters an, beispielsweise in dem man ein inertes Gas durch das Produkt bei erhöhter Temperatur leitet. Es kann auch bei erhöhter Temperatur gleichzeitig ein Unterdruck angelegt werden und gegebenenfalls ein inertes Gas durch das Produkt geleitet werden. Auch ohne Einwirken eines inerten Gases kann nur bei erhöhter Temperatur oder nur bei geringerem Druck gearbeitet werden. Die jeweiligen Trocknungsbedingungen, wie Temperatur, Druck und Dauer lassen sich durch einfache Vorversuche ermitteln. Im Allgemeinen arbeitet man bei Temperaturen im Bereich von 80 bis 250°C, vorzugsweise 100 bis 180°C und bei Drücken von 0,2 bis 500 hPa, vorzugsweise 1 bis 200 hPa und insbesondere 1 bis 20 hPa. Darauf wird der Rohester, falls noch nicht erfolgt, filtriert, um ihn von den Feststoffen, den gegebenenfalls zugesetzten, festen alkalisch reagierenden Stoffen, den Hydrolyseprodukten des Katalysators und dem in der Veresterungsstufe zugesetzten Adsorbens zu befreien. Die Filtration erfolgt in konventionellen Filtrierapparaturen bei normaler Temperatur oder bei Temperaturen bis 120°C. Die Filtration kann durch gängige Filtrierhilfsmittel wie Cellulose, Kieselgel, Kieselgur, Holzmehl, unterstützt werden. Ihr Einsatz ist jedoch auf Ausnahmefälle beschränkt.

Nach Abschluss der Filtration werden hellfarbige Polyolester erhalten, die auch den übrigen Spezifikationen, wie Wassergehalt, Restsäuregehalt, Restgehalt an Katalysatorbestandteilen und Restgehalt an Monoester genügen.

Die als Ausgangsstoffe für das erfindungsgemäße Verfahren eingesetzten mehrwertigen Alkohole oder Polyole genügen der allgemeinen Formel (I)

R(OH)ₙ (I)

in der R einen aliphatischen oder cycloaliphatischen Kohlenwasserstoffrest mit 2 bis 20, vorzugsweise 2 bis 10 Kohlenstoffatomen und n eine ganze Zahl von 2 bis 8, vorzugsweise 2, 3, 4, 5 oder 6 bedeuten.

Als Polyole geeignet sind ebenfalls Verbindungen der allgemeinen Formel (II)

H-(-O-[-CR¹R²-]ₘ-)ₒ-OH (II)

in der R¹ und R² unabhängig voneinander Wasserstoff, einen Alkylrest mit 1 bis 5 Kohlenstoffatomen, vorzugsweise Methyl, Ethyl oder Propyl, oder einen Hydroxyalkylrest mit 1 bis 5 Kohlenstoffatomen, vorzugsweise der Hydroxymethylrest, m eine ganze Zahl von 1 bis 10, vorzugsweise 1 bis 8 und insbesondere 1, 2, 3 oder 4, o eine ganze Zahl von 2 bis 15, vorzugsweise 2 bis 8 und insbesondere 2, 3, 4 oder 5 bedeuten.
Als Polyole, die nach dem erfindungsgemäßen Verfahren zu hellfarbigen Polyolestern umgesetzt werden können, eignen sich beispielsweise 1,3-Propandiol, 1,3-Butandiol, 1,4-Butandiol, Neopentylglykol, 2,2-Dimethylolbutan, Trimethylolethan, Trimethylolpropan, Di-Trimethylolpropan, Trimethylolbutan, 2,2,4-Trimethylpentan-1,3-diol, 1,2-Hexandiol, 1,6-Hexandiol, Pentaerythrit oder Di-Pentaerythrit oder 3(4),8(9)-Dihydroxymethyl-tricyclo[5.2.1.0^{2.6}]decan.

Als weitere Polyole kommen Ethylenglykol und 1,2-Propylenglykol und deren Oligomeren, insbesondere die Etherdiole Di-, Tri- und Tetraethylenglykol oder Dipropylenglykol, Tripropylenglykol oder Tetrapropylenglykol in Betracht. Ethylen- und Propylenglykole sind industriell produzierte Chemikalien. Basissubstanz zu ihrer Herstellung ist Ethylenoxid und Propylenoxid, aus dem man 1,2-Ethylenglykol und 1,2-Propylenglykol durch Erhitzen mit Wasser unter Druck gewinnt. Diethylenglykol erhält man durch Ethoxylierung aus Ethylenglykol. Triethylenglykol fällt, wie Tetraethylenglykol, als Nebenprodukt bei der Hydrolyse von Ethylenoxid zur Herstellung von Ethylenglykol an. Beide Verbindungen können auch durch Umsetzung von Ethylenglykol mit Ethylenoxid synthetisiert werden. Dipropylenglykol, Tripropylenglykol, Tetrapropylenglykol und höhere Propoxylierungsprodukte sind aus der mehrfachen Addition von Propylenoxid an 1,2-Propylenglykol zugänglich.

Zur Gewinnung von hellfarbigen Polyolestern nach dem erfindungsgemäßen Prozess setzt man lineare oder verzweigte, aliphatische Monocarbonsäuren mit 3 bis 20 Kohlenstoffatomen im Molekül ein. Obgleich man gesättigten Säuren in vielen Fällen den Vorzug gibt, können, in Abhängigkeit vom jeweiligen Anwendungsgebiet der Weichmacher oder Schmiermittel, auch ungesättigte Carbonsäuren als Reaktionskomponente zur Estersynthese verwendet werden. Beispiele für Monocarbonsäuren als Bausteine von Polyolestern sind Propionsäure, n-Buttersäure, Isobuttersäure, n-Pentansäure, 2-Methylbuttersäure, 3-Methylbuttersäure, 2-Methylpentansäure, n-Hexansäure, 2-Ethylbuttersäure, n-Heptansäure, 2-Methylhexansäure, Cyclohexancarbonsäure, 2-Ethylhexansäure, n-Nonansäure, 2-Methyloctansäure, Isononansäure, 3,5,5-Trimethylhexansäure, 2-Propylheptansäure, 2-Methylundecansäure, Isoundecancarbonsäure, Tricyclodecancarbonsäure und Isotridecancarbonsäure. Besonders bewährt hat sich das neue Verfahren für die Herstellung von Polyolestern des Monoethylenglykols bzw. der oligomeren Ethylenglykole sowie des 1,2-Propylenglykols bzw. der oligomeren Propylenglykole mit C₄- bis C₁₃- bzw. C₅- bis C₁₀-Monocarbonsäuren sowie zur Herstellung von Polyolestern auf Basis von 1,3-Butandiol, Neopentylglykol, 2,2,4-Trimethylpentan-1,3-diol, Trimethylolpropan, Di-Trimethylolpropan, Pentaerythrit oder 3(4),8(9)-Dihydroxymethyl-tricyclo[5.2.1.0^{2.6}]decan.

Die Polyolester des Ethylenglykols sowie seine Oligomeren eignen sich hervorragend als Weichmacher für alle gängigen hochpolymeren thermoplastischen Stoffe. Besonders bewährt haben sie sich als Zusatz zu Polyvinylbutyral, das mit Glykolestern versetzt als Zwischenschicht zur Herstellung von Mehrschichten- oder Verbundgläsern verwendet wird. Sie können ebenfalls als Koaleszenzmittel oder Filmbildehilfsmittel in wässrigen Dispersionen von Kunststoffen, die als Beschichtungsmittel vielfältige Anwendung finden, eingesetzt werden. Nach dem erfindungsgemäßen Herstellungsverfahren lassen sich auf einfache Weise Polyolester mit ausgezeichneten Farbeigenschaften herstellen, die auch weiteren Qualitätsansprüchen, wie geringem Geruch oder einer geringen Säurezahl genügen. Besonders eignet sich das erfindungsgemäße Verfahren zur Herstellung von Triethylenglykol-di-2-ethyl-hexanoat (3G8 Ester), Tetraethylenglykol-di-n-heptanoat (4G7 Ester), Triethylenglykol-di-2-ethylbutyrat (3G6 Ester), Triethylenglykol-di-n-heptanoat (3G7 Ester) oder Tetraethylenglykol-di-2-ethylhexanoat (4G8 Ester).

Das erfindungsgemäße Verfahren kann kontinuierlich oder absatzweise in den für die chemische Technik typischen Reaktionsapparaten durchgeführt werden. Bewährt haben sich Rührkessel oder Reaktionsrohre, wobei die absatzweise Reaktionsführung die bevorzugte ist.

In den folgenden Beispielen wird das erfindungsgemäße Verfahren näher erläutert, es ist jedoch nicht auf die beschriebene Ausführungsform beschränkt.

### Ausführungsbeispiele

### Beispiel 1:

### Herstellung von Triethylenglykol-di-2-ethylhexanoat (3G8 Ester)

Die Veresterung von Triethylenglykol mit 2-Ethylhexansäure wurde in einem beheizbaren 2 l - Vierhalskolben durchgeführt, der mit Rührer, Innenthermometer und einem Wasserabscheider ausgestattet wurde.

In dem Kolben wurden Triethylenglykol und 2-Ethylhexansäure im 20 mol%igem Überschuss, bezogen auf die zu veresternde Hydroxylgruppe, und 1,8 mol-% Tetra(isopropyl)orthotitanat, bezogen auf Triethylenglykol, vorgelegt. Unter Rühren und unter Anlegen eines Unterdrucks bis 600 hPa wurde der Ansatz auf 190°C erhitzt und gebildetes Reaktionswasser an dem Wasserabscheider abgenommen. Bei diesem und den folgenden Beispielen wurde das Auftreten des ersten Reaktionswassers als Startpunkt für das Festlegen der Reaktionszeit gewählt. Nach einer Reaktionszeit von 2 Stunden bei dieser Stufe wurde der Druck auf 400 hPa erniedrigt und die Temperatur auf 220°C erhöht. Der Reaktionsverlauf wurde durch kontinuierliche Auswaage des über den Wasserabscheider ausgeschleusten Reaktionswassers sowie durch Probenahme und gaschromatographische Untersuchung der Proben verfolgt. Bei einem gaschromatographisch ermittelten Gehalt (Gew.-%) an Triethylenglykol-di-2-ethylhexanoat von mindestens 97% sowie bei einer Resthydroxylzahl von maximal 5,0 mg KOH/g (nach DIN 53240) wurde die Reaktion durch Abkühlen des Ansatzes beendet. Die Veresterungsdauer betrug 6 Stunden.

### Beispiel 2:

### Herstellung von Triethylenglykol-di-2-ethylhexanoat (3G8 Ester), Zusatz von Aktivkohle während der Veresterung

Beispiel 2 wurde in gleicher Weise wie Beispiel 1 durchgeführt, mit der einzigen Ausnahme, dass man zu Beginn der Veresterungsreaktion 0,3 Gew.-% Aktivkohle, bezogen auf den gesamten Reaktionsansatz, zusetzte. Nach Erreichen der gemäß Beispiel 1 angegebenen Kennzahlen, die den Veresterungsgrad charakterisieren, wurde die Reaktion beendet. Die Veresterungsdauer betrug 6 Stunden.

### Beispiel 3:

### Herstellung von Triethylenglykol-di-2-ethylhexanoat (3G8 Ester), Zusatz von Aktivkohle während der Veresterung

In einem beheizbaren 2 l-Vierhalskolben, versehen mit Rührer, Innenthermometer und Wasserabscheider wurden Triethylenglykol und 2-Ethylhexansäure im 30 mol-%igem Überschuss, bezogen auf die zu veresternde Hydroxylgruppe, und 0,018 mol-% Tetra(isopropyl)orthotitanat, bezogen auf Triethylenglykol, vorgelegt und mit 1 Gew.-% Aktivkohle, bezogen auf den gesamten Reaktionsansatz, versetzt. Unter Rühren und unter Anlegen eines Unterdrucks bis 600 hPa wurde der Ansatz auf 220°C erhitzt und gebildetes Reaktionswasser an dem Wasserabscheider abgenommen. Nach einer Reaktionszeit von 1 Stunden bei dieser Stufe wurde der Druck auf 400 hPa erniedrigt und die Temperatur bei 220°C belassen. Nach weiteren 3 Stunden Reaktionszeit wurde der Druck auf 300 hPa weiter reduziert. Der Reaktionsverlauf wurde durch kontinuierliche Auswaage des über den Wasserabscheider ausgeschleusten Reaktionswassers sowie durch Probenahme und gaschromatographische Untersuchung der Proben verfolgt. Bei einem gaschromatographisch ermittelten Gehalt (Gew.-%) an Triethylenglykol-di-2-ethylhexanoat von mindestens 97% sowie bei einer Resthydroxylzahl von maximal 5,0 mg KOH/g (nach DIN 53240) wurde die Reaktion durch Abkühlen des Ansatzes beendet. Die Veresterungsdauer betrug 8 Stunden.

### Beispiel 4:

### Herstellung von Triethylenglykol-di-2-ethylhexanoat (3G8 Ester), Zusatz von Aktivkohle während der Veresterung; Zinn-Katalyse

In einem beheizbaren 2 l-Vierhalskolben, versehen mit Rührer, Innenthermometer und Wasserabscheider wurden Triethylenglykol und 2-Ethylhexansäure im 30 mol-%igem Überschuss, bezogen auf die zu veresternde Hydroxylgruppe, und 0,36 mol-% Zinn(II)-2-ethylhexanoat, bezogen auf Triethylenglykol, vorgelegt und mit 0,3 Gew.-% Aktivkohle, bezogen auf den gesamten Reaktionsansatz, versetzt. Unter Rühren und unter Anlegen eines Unterdrucks bis 600 hPa wurde der Ansatz auf 220°C erhitzt und gebildetes Reaktionswasser an dem Wasserabscheider abgenommen. Nach einer Reaktionszeit von 2 Stunden bei dieser Stufe wurde der Druck auf 400 hPa erniedrigt und die Temperatur bei 220°C belassen. Nach weiteren 4 Stunden Reaktionszeit wurde der Druck auf 300 hPa weiter reduziert. Der Reaktionsverlauf wurde durch kontinuierliche Auswaage des über den Wasserabscheider ausgeschleusten Reaktionswassers sowie durch Probenahme und gaschromatographische Untersuchung der Proben verfolgt. Bei einem gaschromatographisch ermittelten Gehalt (Gew.-%) an Triethylenglykol-di-2-ethylhexanoat von mindestens 97% sowie bei einer Resthydroxylzahl von maximal 5,0 mg KOH/g (nach DIN 53240) wurde die Reaktion durch Abkühlen des Ansatzes beendet. Die Veresterungsdauer betrug 6 Stunden.

### Beispiel 5:

### Herstellung von Triethylenglykol-di-2-ethylhexanoat (3G8 Ester), Zusatz von Aktivkohle während der Veresterung, Zink-Katalyse.

In einem beheizbaren 2 l - Vierhalskolben, versehen mit Rührer, Innenthermometer und Wasserabscheider wurden Triethylenglykol und 2-Ethylhexansäure im 30%igem Überschuss, bezogen auf die zu veresternde Hydroxylgruppe, und 0,36 mol-% Zink(II)acetat Dihydrat, bezogen auf Triethylenglykol, vorgelegt und mit 1,0 Gew.-% Aktivkohle, bezogen auf den gesamten Reaktionsansatz, versetzt. Unter Rühren und unter Anlegen eines Unterdrucks bis 600 hPa wurde der Ansatz auf 220°C erhitzt und gebildetes Reaktionswasser an dem Wasserabscheider abgenommen. Nach einer Reaktionszeit von 2 Stunden bei dieser Stufe wurde der Druck auf 400 hPa erniedrigt und die Temperatur bei 220°C belassen. Der Reaktionsverlauf wurde durch kontinuierliche Auswaage des über den Wasserabscheider ausgeschleusten Reaktionswassers sowie durch Probenahme und gaschromatographische Untersuchung der Proben verfolgt. Die Veresterungsdauer betrug 7 Stunden.

Aufarbeitung der Reaktionsansätze nach den Beispielen 1 bis 5 mit der destillativen 2-Ethylhexansäureabtrennung, der Wasserdampfdestillation, der Trocknung und anschließenden Filtration
A) Destillative Abtrennung der überschüssigen 2-Ethylhexansäure bis zu einem Restsäuregehalt im Rohester von < 1 mg KOH/g (DIN EN ISO 3682/ASTM D 1613)

| Rohester gemäß Beispiel | Destillationstemperatur (°) | Destillationsdruck (hPa) |
|---|---|---|
| 1 | 180 | 2 |
| 2 | 180 | 2 |
| 3 | 180 | beginnend bei 300 hPa unter Druckreduzierung auf 2 hPa über 2 Stunden |
| 4 | 200 | beginnend bei 300 hPa unter Druckreduzierung auf 2 hPa über 2 Stunden |
| 5 | 200 | 2 |

B) Wasserdampfdestillation (Dauer jeweils 1 Stunde)

| Rohester gemäß Beispiel | Sumpftemperatur (°) | Destillationsdruck (hPa) |
|---|---|---|
| 1 | 180 | Normaldruck |
| 2 | 180 | Normaldruck |
| 3 | 180 | Normaldruck |
| 4 | 200 | Normaldruck |
| 5 | 180 | Normaldruck / Dauer: 1,5 Stunden |

C) Trocknung (Dauer jeweils 0,5 Stunde)

| Rohester gemäß Beispiel | Sumpftemperatur (°) | Druck (hPa) |
|---|---|---|
| 1 | 140 | 2 |
| 2 | 140 | 2 |
| 3 | 140 | 10 |
| 4 | 160 | 10 / Dauer: 1,5 Stunden |
| 5 | 160 | 10 / Dauer: 1,5 Stunden |

D) Nach Filtration der abgeschiedenen Feststoffe und der zugesetzten Aktivkohle bei Normaldruck und Raumtemperatur erhielt man als Rückstand einen hellfarbigen Polyolester mit folgenden Kennzahlen:
Gaschromatographische Analyse (Gew.-%):

| | Beispiel 1 % | Beispiel 2 % | Beispiel 3 % | Beispiel 4 % | Beispiel 5 % |
|---|---|---|---|---|---|
| Triethylenglykol-di-2-ethyl-hexanoat | 97,4 | 97,6 | 98,2 | 98,2 | 95,5 |
| Triethylenglykolmono-2-ethyl-hexanoat | 0,9 | 0,8 | 0,4 | 0,8 | 2,0 |
| Diethylenglykol-di-2-ethyl-hexanoat | 0,4 | 0,5 | 0,4 | 0,3 | 0,6 |
| Rest | 1,3 | 1,1 | 1,0 | 0,7 | 1,9 |

Kennzahlen:

| | Beispiel 1 | Beispiel 2 | Beispiel 3 | Beispiel 4 | Beispiel 5 |
|---|---|---|---|---|---|
| Farbzahl nach Hazen (DIN ISO 6271) | 28 | 23 | 16 | 27 | 89 |
| Neutralisationszahl (mg KOH/g, DIN EN ISO 3682/ ASTM D 1613) | 0,07 | 0,09 | 0,10 | 0,08 | 0,29 |
| Wassergehalt (Gew.-%, DIN 51777 Teil 1) | 0,01 | 0,03 | 0,02 | 0,02 | 0,25 |
| Hydroxylzahl (mg KOH/g; DIN 53240) | 2,3 | 2,0 | 1,4 | 1,3 | 3,9 |
| Metallgehalt ppm (DIN EN ISO 11885) | <0,08 | <0,08 | 0,27 | 0,27 | 7,9 |

### Beispiel 6:

### Herstellung von Neopentylglykol-di-2-ethylhexanoat, Zusatz von Aktivkohle während der Veresterung

In einem beheizbaren 2 l-Vierhalskolben, versehen mit Rührer, Innenthermometer und Wasserabscheider wurden Neopentylglykol und 2-Ethylhexansäure im 30 mol-%igem Überschuss, bezogen auf die zu veresternde Hydroxylgruppe, und 0,36 mol-% Tetra(isopropyl)orthotitanat, bezogen auf Neopentylglykol, vorgelegt und mit 1 Gew.-% Aktivkohle, bezogen auf den gesamten Reaktionsansatz, versetzt. Unter Rühren und unter Anlegen eines Unterdrucks bis 600 hPa wurde der Ansatz auf 200°C erhitzt und gebildetes Reaktionswasser an dem Wasserabscheider abgenommen. Nach einer Reaktionszeit von 2 Stunden bei dieser Stufe wurde der Druck auf 450 hPa erniedrigt und die Temperatur bei 200°C belassen. Der Reaktionsverlauf wurde durch kontinuierliche Auswaage des über den Wasserabscheider ausgeschleusten Reaktionswassers sowie durch Probenahme und gaschromatographische Untersuchung der Proben verfolgt. Bei einem gaschromatographisch ermittelten Gehalt (Gew.-%) an Neopentylglykol-di-2-ethylhexanoat von mindestens 97% sowie bei einer Resthydroxylzahl von maximal 5,0 mg KOH/g (nach DIN 53240) wurde die Reaktion durch Abkühlen des Ansatzes beendet. Die Veresterungsdauer betrug 7 Stunden.

Im Anschluss daran wurde zunächst die überschüssige 2-Ethylhexansäure bei einer Temperatur von 150°C und bei einem Druck von 5 hPa abdestilliert. Es folgte eine Wasserdampfdestillation bei einer Sumpftemperatur von 200°C über einen Zeitraum von 1,5 Stunden und dann eine Trocknung bei einer Temperatur von 160°C und bei einem Druck von 10 hPa über einen Zeitraum von 2 Stunden. Nach Filtration der abgeschiedenen Feststoffe und des zugesetzten Adsorbens erhielt man als Rückstand einen hellfarbigen Polyolester mit folgenden Kennzahlen:

### Gaschromatographische Analyse (Gew.-%):

| | |
|---|---|
| Neopentylglykol-di-2-ethylhexanoat | 96,3 |
| Neopentylglykol-mono-2-ethylhexanoat | 2,5 |
| Rest | 1,2 |

### Kennzahlen:

| | |
|---|---|
| Farbzahl nach Hazen (DIN ISO 6271) | 32 |
| Neutralisationszahl (mg KOH/g, DIN EN ISO 3682/ ASTM D 1613) | 1,21 |
| Wassergehalt (Gew.-%, DIN 51777 Teil 1) | 0,1 |
| Hydroxylzahl (mg KOH/g; DIN 53240) | 4,5 |
| Metallgehalt ppm (DIN EN ISO 11885) | 0,64 |

Durch die erfindungsgemäßen Maßnahmen, sowohl die Veresterungsstufe als auch die Aufarbeitungsstufe gezielt aufeinander abzustimmen, lassen sich Polyolester mit hervorragender Qualität erhalten, die ihren Einsatz in einer Vielzahl von Anwendungen ermöglicht.

### Beispiel 7 (Vergleich):

### Herstellung von Triethylenglykol-di-2-ethylhexanoat (3G8 Ester), Zinn-Katalyse

Die Veresterung von Triethylenglykol mit 2-Ethylhexansäure wurde in einem beheizbaren 2 l - Vierhalskolben durchgeführt, der mit Rührer, Innenthermometer und einem Wasserabscheider ausgestattet wurde.

In dem Kolben wurden Triethylenglykol und 2-Ethylhexansäure im 30 mol%igem Überschuss, bezogen auf die zu veresternde Hydroxylgruppe, und 1,8 mol-% Zinn(II)-2-ethylhexanoat, bezogen auf Triethylenglykol, vorgelegt. Unter Rühren und unter Anlegen eines Unterdrucks bis 600 hPa wurde der Ansatz auf 220°C erhitzt und gebildetes Reaktionswasser an dem Wasserabscheider abgenommen. Nach einer Reaktionszeit von 2 Stunden bei dieser Stufe wurde der Druck auf 400 hPa erniedrigt. Der Reaktionsverlauf wurde durch kontinuierliche Auswaage des über den Wasserabscheider ausgeschleusten Reaktionswassers sowie durch Probenahme und gaschromatographische Untersuchung der Proben verfolgt. Die Veresterung wurde nach 5 Stunden abgebrochen.

In diesem Beispiel wird das Maximum von Triethylenglykol-di-2-ethylhexanoat bereits nach 2 Stunden erreicht und das rohe Reaktionsgemisch weist folgende gaschromatographisch ermittelte Zusammensetzung auf (2-Ethylhexansäure-frei gerechnet):

| | |
|---|---|
| Triethylenglykol-di-2-ethylhexanoat | 93,5 Gew.-% |
| Triethylenglykol-mono-2-ethylhexanoat | 2,3 Gew.-% |
| Diethylenglykol-di-2-ethylhexanoat | 0,6 Gew.-% |
| Rest | 3,6 Gew.-% |

Nach 5 Stunden wurde die Reaktion beendet. Nach Abtrennung überschüssiger 2-Ethylhexansäure weist der rohe Polyolester folgende gaschromatographisch ermittelte Zusammensetzung auf:

| | |
|---|---|
| Triethylenglykol-di-2-ethylhexanoat | 86,2 Gew.-% |
| Triethylenglykol-mono-2-ethylhexanoat | 0,6 Gew.-% |
| Diethylenglykol-di-2-ethylhexanoat | 3,3 Gew.-% |
| Rest | 9,9 Gew.-% |

Die übrigen Kennzahlen wurden nicht bestimmt.

Wie die Ergebnisse des Beispiels 7 zeigen, wird bereits nach 2 Stunden Veresterungsdauer das Maximum an Triethylenglykol-di-2-ethylhexanoat erreicht. Bei der dann folgenden Restumsatzphase tritt überwiegend die Spaltung der Etherkette unter Zunahme des Gehaltes an Diethylenglykol-di-2-ethylhexanoat sowie an Restkomponenten ein.

Gegenüber Beispiel 4 belegt dieses Vergleichsbeispiel, dass bei hohen Katalysatorkonzentrationen mit einem optimierten Temperatur- und Druck-profil zu arbeiten ist, um Polyolester in ausreichender Qualität zu gewinnen.

## Patentansprüche

1. Verfahren zur Herstellung von Polyolestern durch Umsetzung von Polyolen mit linearen oder verzweigten aliphatischen Monocarbonsäuren mit 3 bis 20 Kohlenstoffatomen, **dadurch gekennzeichnet, dass** man eine Mischung der Ausgangsverbindungen in Gegenwart einer Lewissäure enthaltend mindestens ein Element der Gruppen 4 bis 14 des Periodensystems der Elemente als Katalysator unter Entfernung des gebildeten Wassers reagieren lässt, wobei die Umsetzung der Ausgangsverbindungen in Gegenwart eines Adsorbens erfolgt, und anschließend eine Wasserdampfbehandlung zur Restsäure- und Wasserentfernung durchführt.

2. Verfahren gemäß Anspruch 1, **dadurch gekennzeichnet, dass** man die Mischung der Ausgangsverbindungen in Gegenwart des Katalysators auf eine Temperatur bis auf maximal 280°C, vorzugsweise bis auf 250°C, erhitzt und bei konstant gehaltener Temperatur den Druck von Stufe zu Stufe erniedrigt.

3. Verfahren gemäß Anspruch 1, **dadurch gekennzeichnet, dass** man die Mischung der Ausgangsverbindungen in Gegenwart des Katalysators bei konstant eingestelltem Druck von Stufe zu Stufe bis auf eine maximale Temperatur von 280°C erhitzt.

4. Verfahren gemäß Anspruch 1, **dadurch gekennzeichnet, dass** man die Mischung der Ausgangsverbindungen in Gegenwart des Katalysators bei von Stufe zu Stufe ansteigender Temperatur auf maximal 280°C erhitzt und auch den Druck von Stufe zu Stufe erniedrigt.

5. Verfahren gemäß Anspruch 4, **dadurch gekennzeichnet, dass** man eine Mischung der Ausgangsverbindungen in Gegenwart des Katalysators in einer ersten Stufe bei einer Temperatur bis auf 190°C und bei einem Druck bis auf 600 hPa reagieren lässt und die Reaktion in einer zweiten Stufe durch Erhöhung der Temperatur bis auf 250°C und bei einem Druck bis auf 300 hPa zu Ende führt.

6. Verfahren gemäß einem oder mehreren der Ansprüche 1 bis 5, **dadurch gekennzeichnet, dass** der Katalysator in einer Menge von 1,0 X 10 ⁻⁵ bis 20 mol-%, bezogen auf die im Unterschuss eingesetzte Ausgangsverbindung, eingesetzt wird.

7. Verfahren gemäß Anspruch 6, **dadurch gekennzeichnet, dass** der Katalysator in einer Menge von 0,01 bis 5, vorzugsweise 0,01 bis 2 mol-%, bezogen auf die im Unterschuss eingesetzte Ausgangsverbindung, eingesetzt wird.

8. Verfahren gemäß einem oder mehreren der Ansprüche 1 bis 7, **dadurch gekennzeichnet, dass** man als Katalysator Titan, Zirkonium, Eisen, Zink, Bor, Aluminium oder Zinn als Elemente oder in Form ihrer Verbindungen verwendet.

9. Verfahren gemäß Anspruch 8, **dadurch gekennzeichnet, dass** man als Zinnverbindungen Zinn(II)-oxid, Zinn(II)-oxalat, Zinn(II)-carboxylate, Zinn(IV)-alkoholate oder Organozinnverbindungen verwendet.

10. Verfahren gemäß Anspruch 8, **dadurch gekennzeichnet, dass** man als Titanverbindungen Alkoholate, Acylate oder Chelate verwendet.

11. Verfahren gemäß Anspruch 8, **dadurch gekennzeichnet, dass** man als Borverbindungen Borsäure oder Borsäureester verwendet.

12. Verfahren gemäß Anspruch 8, **dadurch gekennzeichnet, dass** man als Aluminiumverbindungen Aluminiumoxid, Aluminiumhydroxid, Aluminiumcarboxylate oder Aluminiumalkoholate verwendet.

13. Verfahren gemäß Anspruch 8, **dadurch gekennzeichnet, dass** man als Zinkverbindungen Zinkoxid, Zinksulfat oder Zinkcarboxylate verwendet.

14. Verfahren gemäß einem oder mehreren der Ansprüche 1 bis 13, **dadurch gekennzeichnet, dass** man auf 100 Gewichtsteilen Reaktionsansatz 0,1 bis 5, vorzugsweise 0,1 bis 1,5, Gew.-Teile Adsorbens verwendet.

15. Verfahren gemäß Anspruch 14, **dadurch gekennzeichnet, dass** man als Adsorbens Silicagel, Kieselgel, Kieselguhr, Aluminiumoxid, Aluminiumoxidhydrate, Tone, Carbonate oder Aktivkohle verwendet.

16. Verfahren gemäß einem oder mehreren der Ansprüche 1 bis 15, **dadurch gekennzeichnet, dass** man die Wasserdampfbehandlung bei einer Temperatur von 100 bis 250°C, vorzugsweise von 150 bis 220°C und insbesondere von 170 bis 200°C durchführt.

17. Verfahren gemäß Anspruch 16, **dadurch gekennzeichnet, dass** man nach der Wasserdampfbehandlung einen festen, alkalisch reagierenden Stoff zusetzt.

18. Verfahren gemäß Anspruch 17, **dadurch gekennzeichnet, dass** man als festen, alkalisch reagierenden Stoff basisches Siliziumdioxid, basisches Aluminiumoxid, Natriumcarbonat, Natriumhydrogencarbonat, Calciumcarbonat, Natriumhydroxid oder basisch reagierende Materialien zusetzt.

19. Verfahren gemäß einem oder mehreren der Ansprüche 1 bis 18, **dadurch gekennzeichnet, dass** der Polyolester nach der Wasserdampfbehandlung bei Temperaturen von 80 bis 250°C, vorzugsweise 100 bis 180°C und bei Drücken von 0,2 bis 500 hPa, vorzugsweise 1 bis 200 hPa und insbesondere von 1 bis 20 hPa getrocknet wird.

20. Verfahren gemäß Anspruch 19, **dadurch gekennzeichnet, dass** der Polyolester in Gegenwart eines Inertgases getrocknet wird.

21. Verfahren gemäß einem oder mehreren der Ansprüche 1 bis 20, **dadurch gekennzeichnet, dass** man als Polyole Verbindungen der allgemeinen Formel (I)
R(OH)ₙ (I)
verwendet, in der R einen aliphatischen oder cycloaliphatischen Kohlenwasserstoffrest mit 2 bis 20, vorzugsweise 2 bis 10 Kohlenstoffatomen und n eine ganze Zahl von 2 bis 8, vorzugsweise 2, 3, 4, 5 oder 6, bedeuten.

22. Verfahren gemäß einem oder mehreren der Ansprüche 1 bis 20, **dadurch gekennzeichnet, dass** man als Polyole Verbindungen der allgemeinen Formel (II)
H-(-O-[-CR¹R²-]ₘ-)ₒ-OH (II)
verwendet, in der R¹ und R² unabhängig voneinander Wasserstoff, einen Alkylrest mit 1 bis 5 Kohlenstoffatomen, vorzugsweise Methyl, Ethyl oder Propyl, oder einen Hydroxyalkylrest mit 1 bis 5 Kohlenstoffatomen, vorzugsweise der Hydroxymethylrest, m eine ganze Zahl von 1 bis 10, vorzugsweise 1 bis 8 und insbesondere 1, 2, 3 oder 4, o eine ganze Zahl von 2 bis 15, vorzugsweise 2 bis 8 und insbesondere 2, 3, 4 oder 5 bedeuten.

23. Verfahren gemäß Anspruch 21, **dadurch gekennzeichnet, dass** man als Polyole 1,2-Propandiol, 1,3-Propandiol, 1,3-Butandiol, 1,4-Butandiol, Neopentylglykol, 2,2-Dimethylolbutan, Trimethylolethan, Trimethylolpropan, Trimethylolbutan, 2,2,4-Trimethylpentan-1,3-diol, 1,2-Hexandiol, 1,6-Hexandiol, Pentaerythrit, Ethylenglykol oder 3(4),8(9)-Dihydroxymethyltricyclo[5.2.1.0^{2.6}]decan verwendet.

24. Verfahren gemäß Anspruch 22, **dadurch gekennzeichnet, dass** man als Polyole Di-Trimethylolpropan, Di-Pentaerythrit, Diethylenglykol, Triethylenglykol, Tetraethylenglykol, Dipropylenglykol, Tripropylenglykol oder Tetrapropylenglykol verwendet.

25. Verfahren gemäß einem oder mehreren der Ansprüche 1 bis 24, **dadurch gekennzeichnet, dass** man als aliphatische Monocarbonsäure Propionsäure, n-Buttersäure, Isobuttersäure, n-Pentansäure, 2-Methylbuttersäure, 3-Methylbuttersäure, 2-Methylpentansäure, n-Hexansäure, 2-Ethylbuttersäure, n-Heptansäure, 2-Methylhexansäure, 2-Ethylhexansäure, n-Nonansäure, 2-Methyloctansäure, Isononansäure, 3,5,5-Trimethylhexansäure oder 2-Propylheptansäure umsetzt.

26. Verfahren gemäß einem oder mehreren der Ansprüche 1 bis 25 zur Herstellung von Triethylenglykol-di-2-ethylhexanoat, Tetraethylenglykol-di-n-heptanoat, Triethylenglykol-di-2-ethylbutyrat, Triethylenglykol-di-n-heptanoat oder Tetraethylenglykol-di-2-ethylhexanoat.

## Claims

1. Process for preparing polyol esters by reacting polyols with linear or branched aliphatic monocarboxylic acids having 3 to 20 carbon atoms, **characterized in that** a mixture of the starting compounds is allowed to react in the presence of a Lewis acid containing at least one element of groups 4 to 14 of the Periodic Table of the Elements as a catalyst with removal of the water formed, the starting compounds being converted in the presence of an adsorbent, and then a steam treatment is performed to remove residual acid and water.

2. Process according to Claim 1, **characterized in that** the mixture of the starting compounds is heated in the presence of the catalyst to a temperature up to a maximum of 280°C, preferably up to 250°C, and the pressure is lowered from stage to stage with the temperature kept constant.

3. Process according to Claim 1, **characterized in that** the mixture of the starting compounds is heated in the presence of the catalyst at constant pressure from stage to stage up to a maximum temperature of 280°C.

4. Process according to Claim 1, **characterized in that** the mixture of the starting compounds is heated in the presence of the catalyst at a temperature rising from stage to stage to a maximum of 280°C, and the pressure is also lowered from stage to stage.

5. Process according to Claim 4, **characterized in that** a mixture of the starting compounds is allowed to react in the presence of the catalyst in a first stage at a temperature up to 190°C and at a pressure up to 600 hPa, and the reaction is conducted to completion in a second stage by increasing the temperature up to 250°C and at a pressure up to 300 hPa.

6. Process according to one or more of Claims 1 to 5, **characterized in that** the catalyst is used in an amount of 1.0 x 10⁻⁵ to 20 mol%, based on the starting compound used in deficiency.

7. Process according to Claim 6, **characterized in that** the catalyst is used in an amount of 0.01 to 5 and preferably 0.01 to 2 mol%, based on the starting compound used in deficiency.

8. Process according to one or more of Claims 1 to 7, **characterized in that** the catalyst used is titanium, zirconium, iron, zinc, boron, aluminium or tin in the form of elements or in the form of compounds thereof.

9. Process according to Claim 8, **characterized in that** the tin compounds used are tin(II) oxide, tin(II) oxalate, tin(II) carboxylates, tin(IV) alkoxides or organotin compounds.

10. Process according to Claim 8, **characterized in that** the titanium compounds used are alkoxides, acrylates or chelates.

11. Process according to Claim 8, **characterized in that** the boron compounds used are boric acid or boric esters.

12. Process according to Claim 8, **characterized in that** the aluminium compounds used are aluminium oxide, aluminium hydroxide, aluminium carboxylates or aluminium alkoxides.

13. Process according to Claim 8, **characterized in that** the zinc compounds used are zinc oxide, zinc sulphate or zinc carboxylates.

14. Process according to one or more of Claims 1 to 13, **characterized in that** 0.1 to 5 and preferably 0.1 to 1.5 parts by weight of adsorbent are used per 100 parts by weight of reaction mixture.

15. Process according to Claim 14, **characterized in that** the adsorbent used is silica gel, kieselguhr, aluminium oxide, aluminium oxide hydrates, clays, carbonates or activated carbon.

16. Process according to one or more of Claims 1 to 15, **characterized in that** the steam treatment is performed at a temperature of 100 to 250°C, preferably of 150 to 220°C and especially of 170 to 200°C.

17. Process according to Claim 16, **characterized in that**, after the steam treatment, a solid alkaline substance is added.

18. Process according to Claim 17, **characterized in that** the solid alkaline substance added is basic silicon dioxide, basic aluminium oxide, sodium carbonate, sodium hydrogencarbonate, calcium carbonate, sodium hydroxide or basic minerals.

19. Process according to one or more of Claims 1 to 18, **characterized in that** the polyol ester, after the steam treatment, is dried at temperatures of 80 to 250°C, preferably 100 to 180°C, and at pressures of 0.2 to 500 hPa, preferably 1 to 200 hPa and especially of 1 to 20 hPa.

20. Process according to Claim 19, **characterized in that** the polyol ester is dried in the presence of an inert gas.

21. Process according to one or more of Claims 1 to 20, **characterized in that** the polyols used are compounds of the general formula (I)
R(OH)ₙ (I)
in which R is an aliphatic or cycloaliphatic hydrocarbon radical having 2 to 20 and preferably 2 to 10 carbon atoms, and n is an integer of 2 to 8, preferably 2, 3, 4, 5 or 6.

22. Process according to one or more of Claims 1 to 20, **characterized in that** the polyols used are compounds of the general formula (II)
H- (-O-[-CR¹R²-]ₘ-)ₒ-OH (II)
in which R¹ and R² are each independently hydrogen, an alkyl radical having 1 to 5 carbon atoms, preferably methyl, ethyl or propyl, or a hydroxyalkyl radical having 1 to 5 carbon atoms, preferably the hydroxymethyl radical, m is an integer of 1 to 10, preferably 1 to 8 and especially 1, 2, 3 or 4, o is an integer of 2 to 15, preferably 2 to 8 and especially 2, 3, 4 or 5.

23. Process according to Claim 21, **characterized in that** the polyols used are 1,2-propanediol, 1,3-propanediol, 1,3-butanediol, 1,4-butanediol, neopentyl glycol, 2,2-dimethylolbutane, trimethylolethane, trimethylolpropane, trimethylolbutane, 2,2,4-trimethylpentane-1,3-diol, 1,2-hexanediol, 1,6-hexanediol, pentaerythritol, ethylene glycol or 3(4),8(9)-dihydroxymethyltricyclo[5.2.1.0^{2.6}]decane.

24. Process according to Claim 22, **characterized in that** the polyols used are ditrimethylolpropane, dipentaerythritol, diethylene glycol, triethylene glycol, tetraethylene glycol, dipropylene glycol, tripropylene glycol or tetrapropylene glycol.

25. Process according to one or more of Claims 1 to 24, **characterized in that** the aliphatic monocarboxylic acid converted is propionic acid, n-butyric acid, isobutyric acid, n-pentanoic acid, 2-methylbutyric acid, 3-methylbutyric acid, 2-methylpentanoic acid, n-hexanoic acid, 2-ethyl-butyric acid, n-heptanoic acid, 2-methylhexanoic acid, 2-ethylhexanoic acid, n-nonanoic acid, 2-methyloctanoic acid, isononanoic acid, 3,5,5-trimethylhexanoic acid or 2-propylheptanoic acid.

26. Process according to one or more of Claims 1 to 25 for preparing triethylene glycol di-2-ethyl-hexanoate, tetraethylene glycol di-n-heptanoate, triethylene glycol di-2-ethylbutyrate, triethylene glycol di-n-heptanoate or tetraethylene glycol di-2-ethylhexanoate.

## Revendications

1. Procédé pour la préparation de polyolesters par transformation de polyols avec des acides monocarboxyliques aliphatiques, linéaires ou ramifiés, comprenant 3 à 20 atomes de carbone, **caractérisé en ce qu'**on laisse réagir un mélange des composés de départ en présence d'un acide de Lewis contenant au moins un élément des groupes 4 à 14 du système périodique des éléments comme catalyseur, avec élimination de l'eau formée, la transformation des composés de départ ayant lieu en présence d'un adsorbant, puis on réalise un traitement à la vapeur d'eau pour l'élimination de l'acide résiduel et de l'eau.

2. Procédé selon la revendication 1, **caractérisé en ce qu'**on chauffe le mélange des composés de départ en présence du catalyseur à une température d'au maximum jusqu'à 280°C, de préférence jusqu'à 250°C, et on abaisse, en maintenant la température constante, la pression d'une étape à l'autre.

3. Procédé selon la revendication 1, **caractérisé en ce qu'**on chauffe le mélange des composés de départ en présence du catalyseur à une pression réglée à une valeur constante d'une étape à l'autre jusqu'à une température maximale de 280°C.

4. Procédé selon la revendication 1, **caractérisé en ce qu'**on chauffe le mélange des composés de départ en présence du catalyseur à une température augmentant d'une étape à l'autre d'au maximum à 280°C et on abaisse également la pression d'une étape à l'autre.

5. Procédé selon la revendication 4, **caractérisé en ce qu'**on laisse réagir un mélange des composés de départ en présence du catalyseur dans une première étape à une température de jusqu'à 190°C et à une pression de jusqu'à 600 hPa et on conduit la réaction jusqu'à la fin dans une deuxième étape par augmentation de la température jusqu'à 250°C et à une pression jusqu'à 300 hPa.

6. Procédé selon l'une ou plusieurs des revendications 1 à 5, **caractérisé en ce que** le catalyseur est utilisé en une quantité de 1, 0 X 10⁻⁵ à 20% en mole, par rapport au composé de départ utilisé à une quantité inférieure à la quantité stoechiométrique.

7. Procédé selon la revendication 6, **caractérisé en ce que** le catalyseur est utilisé en une quantité de 0,01 à 5, de préférence de 0,01 à 2% en mole, par rapport au composé de départ utilisé à une quantité inférieure à la quantité stoechiométrique.

8. Procédé selon l'une ou plusieurs des revendications 1 à 7, **caractérisé en ce qu'**on utilise, comme catalyseur, le titane, le zirconium, le fer, le zinc, le bore, l'aluminium ou l'étain en tant qu'éléments ou sous forme de leurs composés.

9. Procédé selon la revendication 8, **caractérisé en ce qu'**on utilise, comme composés de l'étain, l'oxyde d'étain (II), l'oxalate d'étain (II), des carboxylates d'étain (II), des alcoolates d'étain (IV) ou des composés organostanniques.

10. Procédé selon la revendication 8, **caractérisé en ce qu'**on utilise, comme composés du titane, des alcoolates, des acylates ou des chélates.

11. Procédé selon la revendication 8, **caractérisé en ce qu'**on utilise, comme composés du bore, l'acide borique ou des esters de l'acide borique.

12. Procédé selon la revendication 8, **caractérisé en ce qu'**on utilise, comme composés d'aluminium, l'oxyde d'aluminium, l'hydroxyde d'aluminium, des carboxylates d'aluminium ou des alcoolates d'aluminium.

13. Procédé selon la revendication 8, **caractérisé en ce qu'**on utilise, comme composés du zinc, l'oxyde de zinc, le sulfate de zinc ou des carboxylates de zinc.

14. Procédé selon l'une ou plusieurs des revendications 1 à 13, **caractérisé en ce qu'**on utilise, pour 100 parties en poids de charge réactionnelle, 0,1 à 5, de préférence 0,1 à 1,5, parties en poids d'adsorbant.

15. Procédé selon la revendication 14, **caractérisé en ce qu'**on utilise comme adsorbant, le silicagel, le gel silicique, le kieselguhr, l'oxyde d'aluminium, l'oxyde d'aluminium hydraté, l'argile, des carbonates ou le charbon actif.

16. Procédé selon l'une ou plusieurs des revendications 1 à 15, **caractérisé en ce qu'**on réalise le traitement à la vapeur d'eau à une température de 100 à 250°C, de préférence de 150 à 220°C et en particulier de 170 à 200°C.

17. Procédé selon la revendication 16, **caractérisé en ce qu'**on ajoute, après le traitement à la vapeur d'eau, une substance solide, à réaction alcaline.

18. Procédé selon la revendication 17, **caractérisé en ce qu'**on ajoute, comme substance solide à réaction alcaline, le dioxyde de silicium basique, l'oxyde d'aluminium basique, le carbonate de sodium, l'hydrogénocarbonate de sodium, le carbonate de calcium, l'hydroxyde de sodium ou des matériaux à réaction basique.

19. Procédé selon l'une ou plusieurs des revendications 1 à 18, **caractérisé en ce que** le polyolester est séché après le traitement à la vapeur d'eau à des températures de 80 à 250°C, de préférence de 100 à 180°C et à des pressions de 0,2 à 500 hPa, de préférence de 1 à 200 hPa et en particulier de 1 à 20 hPa.

20. Procédé selon la revendication 19, **caractérisé en ce que** le polyolester est séché en présence d'un gaz inerte.

21. Procédé selon l'une ou plusieurs des revendications 1 à 20, **caractérisé en ce qu'**on utilise, comme polyols, des composés de formule générale (I)
R(OH)ₙ (I)
dans laquelle R signifie un radical hydrocarboné aliphatique ou cycloaliphatique comprenant 2 à 20, de préférence 2 à 10 atomes de carbone et n vaut un nombre entier de 2 à 8, de préférence 2, 3, 4, 5 ou 6.

22. Procédé selon l'une ou plusieurs des revendications 1 à 20, **caractérisé en ce qu'**on utilise, comme polyols, des composés de formule générale (II)
H-(-O-[-CR¹R²-]ₘ-)ₒ-OH (II)
dans laquelle R¹ et R² signifient, indépendamment l'un de l'autre, hydrogène, un radical alkyle comprenant 1 à 5 atomes de carbone, de préférence méthyle, éthyle ou propyle, ou un radical hydroxyalkyle comprenant 1 à 5 atomes de carbone, de préférence le radical hydroxyméthyle, m vaut un nombre entier de 1 à 10, de préférence de 1 à 8 et en particulier 1, 2, 3 ou 4, o vaut un nombre entier de 2 à 15, de préférence de 2 à 8 et en particulier 2, 3, 4 ou 5.

23. Procédé selon la revendication 21, **caractérisé en ce qu'**on utilise, comme polyols, le 1,2-propanediol, le 1,3-propanediol, le 1,3-butanediol, le 1,4-butanediol, le néopentylglycol, le 2,2-diméthylolbutane, le triméthyloléthane, le triméthylolpropane, le triméthylolbutane, le 2,2,4-triméthylpentane-1,3-diol, le 1,2-hexanediol, le 1,6-hexanediol, le pentaérythritol, l'éthylèneglycol ou le 3(4),8(9)-dihydroxyméthyltricyclo[5,2,1,0^{2.6}]décane.

24. Procédé selon la revendication 22, **caractérisé en ce qu'**on utilise, comme polyols, le di-triméthylolpropane, le di-pentaérythritol, le diéthylèneglycol, le triéthylèneglycol, le tétraéthylèneglycol, le dipropylèneglycol, le tripropylèneglycol ou le tétrapropylèneglycol.

25. Procédé selon l'une ou plusieurs des revendications 1 à 24, **caractérisé en ce qu'**on utilise comme acide monocarboxylique aliphatique les acides propionique, n-butyrique, isobutyrique, n-pentanoïque, 2-méthylbutyrique, 3-méthylbutyrique, 2-méthylpentanoïque, n-hexanoïque, 2-éthylbutyrique, n-heptanoïque, 2-méthylhexanoïque, 2-éthylhexanoïque, n-nonanoïque, 2-méthyloctanoïque, isononanoïque, 3,5,5-triméthylhexanoïque ou 2-propylheptanoïque.

26. Procédé selon l'une ou plusieurs des revendications 1 à 25 pour la préparation de di-2-éthylhexanoate de triéthylèneglycol, de di-n-heptanoate de tétraéthylèneglycol, de di-2-éthylbutyrate de triéthylèneglycol, de di-n-heptanoate de triéthylèneglycol ou de di-2-éthylhexanoate de tétraéthylèneglycol.
